# EUROPEAN PATENT APPLICATION

(11) **EP 2 343 006 A1**
(43) Date of publication of application: **13.07.2011**
(21) Application number: 10196470.8
(22) Date of filing: 22.12.2010
(51) Int. Cl.: A61B 1/04, A61B 1/07, A61B 5/00

(54) **Medical apparatus and endoscope apparatus**

(30) Priority: 08.01.2010 JP 2010003388; 25.05.2010 JP 2010119746
(71) Applicant: Fujifilm Corporation, Minato-ku Tokyo (JP)
(72) Inventor: ERIKAWA, Akihiko, Kanagawa (JP)
(74) Representative: Klunker . Schmitt-Nilson . Hirsch

(57) **Abstract**

A medical apparatus includes a plurality of illumination windows which are disposed in a tip portion of an insertion unit to be inserted into a subject body and which emit light beams supplied from a plurality of light sources and having different spectra toward the subject body. The plurality of illumination windows include: a pair of first illumination windows; and a pair of second illumination windows which are disposed inside the pair of first illumination windows, respectively, and emit light beams having a shorter wavelength than light beams emitted from the pair of first illumination windows. The medical apparatus further includes an observation window through which to observe the subject body.

## Description

### BACKGROUND OF INVENTION

### Field of the Invention

The present invention relates to a medical apparatus and an endoscope apparatus.

### Description of the Related Art

In enlarged observation using an endoscope, shooting is performed with the distance between the tip of the endoscope and a subject set at about 1 to 3 mm and, in many cases, the angle of view of the endoscope is set at about 50° to 60° (it is set at about 120° to 140° in ordinary observation).

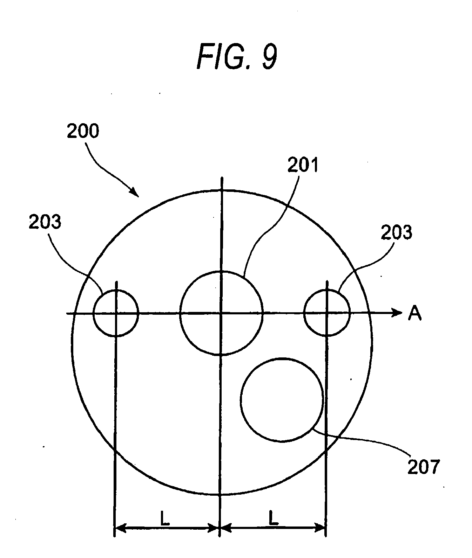

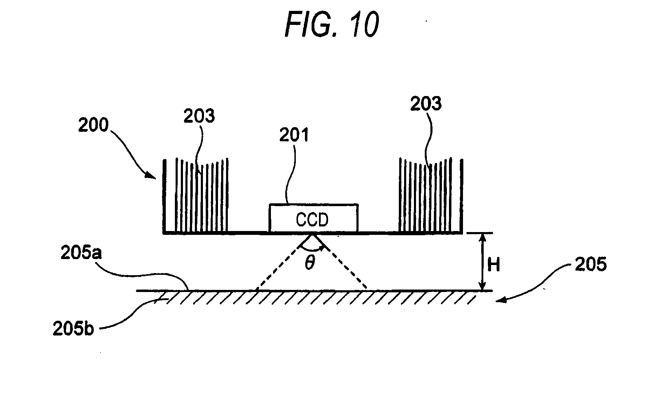

Figs. 9 and 10 are a front view and a sectional view, respectively, of a tip portion of an endoscope 200. When close-proximity shooting is performed on a subject having a distance H from an observation window 201 having an imaging device (CCD (charge-coupled device) image sensor) using the endoscope 200 which is configured as shown in Figs. 9 and 10, the amount of light that reaches a central portion of the imaging surface is smaller than the amount of light that reaches its peripheral portion, as a result of which light quantity unevenness occurs in an observation image. For reasons described below, this light quantity unevenness depends on the color.

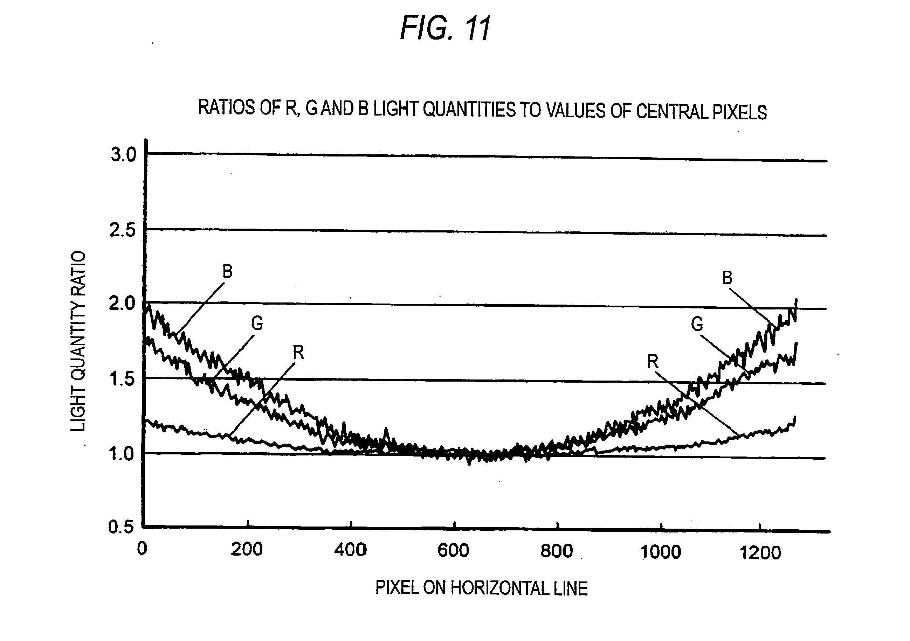

Of the light that is emitted from light guides 203 that are disposed on the two respective sides of the observation window 201 at the same distance L from the observation window 201, a red component having relatively long wavelengths is not only reflected by a surface 205a of a subject (a part of a living body) 205 but also scattered by an inside portion 205b of the subject and scattered light reaches the central portion of the imaging surface. On the other hand, blue light having relatively short wavelengths attenuates immediately in the inside portion 205b of the subject. Therefore, only part of the blue light that is directly reflected by the surface 205a of the subject reaches the central portion of the imaging surface. Therefore, an image taken by the CCD image sensor which is disposed inside the observation window has light quantity ratios shown in the graph of Fig. 11 which are taken along the direction indicated by arrow A in Fig. 9.

In the case of the red component R having relatively long wavelengths, the light quantity ratio is approximately equal to 1.0 anywhere in the horizontal pixel line and the curve is generally flat, that is, the degree of light quantity unevenness is low. On the other hand, in the cases of the green component G and the blue component B having relatively short wavelengths, light quantity values in peripheral regions (near the light guides 203) are larger than light quantity values at the center of the horizontal pixel line. That is, as the wavelength becomes shorter, the light quantity ratio increases more steeply toward the periphery and the degree of light quantity unevenness becomes higher. In Fig. 9, reference numeral 207 denotes a circular forceps hole.

JP-A-2009-34224, for example, discloses a medical apparatus in which an excitation light illumination window is provided separately from an illumination window through which ordinary mode or fluorescence mode illumination light is emitted and a synchronized dimming control is performed so as to prevent illumination light beams emitted from the illumination windows from causing color unevenness. However, this technique does not solve the problem of light quantity differences of each wavelength component.

### SUMMARY OF INVENTION

The present invention has been made in the above circumstances, and an object of the invention is therefore to provide a medical apparatus and an endoscope apparatus which may reduce the degree of light quantity unevenness in close-proximity shooting.
(1) According to an aspect of the invention, a medical apparatus includes a plurality of illumination windows which are disposed in a tip portion of an insertion unit to be inserted into a subject body and which emit light beams supplied from a plurality of light sources and having different spectra toward the subject body, the plurality of illumination windows include: a pair of first illumination windows; and a pair of second illumination windows which are disposed inside the pair of first illumination windows, respectively, and emit light beams having a shorter wavelength than light beams emitted from the pair of first illumination windows; and an observation window through which to observe the subject body.
(2) According to an aspect of the invention, an endoscope apparatus which is a medical apparatus of (1), wherein a plurality of illumination windows and an observation window are disposed in a tip portion of an insertion unit of an endoscope to be inserted into the subject body.

In the medical apparatus and the endoscope apparatus according to the invention, since the illumination windows for emitting shorter wavelength illumination light are disposed closer to the observation window than the other illumination windows are, light quantity unevenness that occurs depending on the observation wavelength in close-proximity observation may be reduced. As a result, a good observation image may be obtained and the accuracy of a diagnosis may be increased.

### BRIEF DESCRIPTION OF THE DRAWINGS

Fig. 1 is a conceptual block diagram showing the configuration of an endoscope apparatus according to an embodiment of the present invention,
Fig. 2 shows an appearance of an example of the endoscope apparatus of Fig. 1,
Fig. 3A is a sectional view showing the structure of a light output unit incorporating a phosphor, and Fig. 3B is a sectional view showing the structure of a light output unit incorporating a light deflecting/diffusing member,
Fig. 4 is a graph showing emission spectra of blue laser light emitted from a laser light source and light generated through wavelength conversion of the blue laser light by the phosphor,
Fig. 5A is a front view of an endoscope tip portion in which plural illumination windows are arranged in line, Fig. 5B is a front view of an endoscope tip portion in which pairs of illumination windows for emitting light beams having the same spectrum are arranged on parallel lines, and Fig. 5C is a front view of an endoscope tip portion in which pairs of illumination windows for emitting light beams having the same spectrum are arranged on intersecting lines,
Fig. 6 is a time chart showing emission timing of shorter wavelength light and longer wavelength light,
Fig. 7 is a conceptual front view of an endoscope using LEDs as light sources,
Fig. 8 is a conceptual block diagram showing the configuration of an endoscope apparatus in which LEDs are used as light sources,
Fig. 9 is a conceptual block diagram showing the configuration of another endoscope apparatus in which LEDs are used as light sources,
Fig. 10 is a sectional view showing a positional relationship between the tip portion of the endoscope of Fig. 9 and a subject in close-proximity shooting, and
Fig. 11 is a graph showing relationships between the position in a pixel line and the ratios of R, G, and B light quantities to values of central pixels.

### DESCRIPTION OF EXEMPLARY EMBODIMENTS

An embodiment of the present invention will be hereinafter described with reference to the drawings.

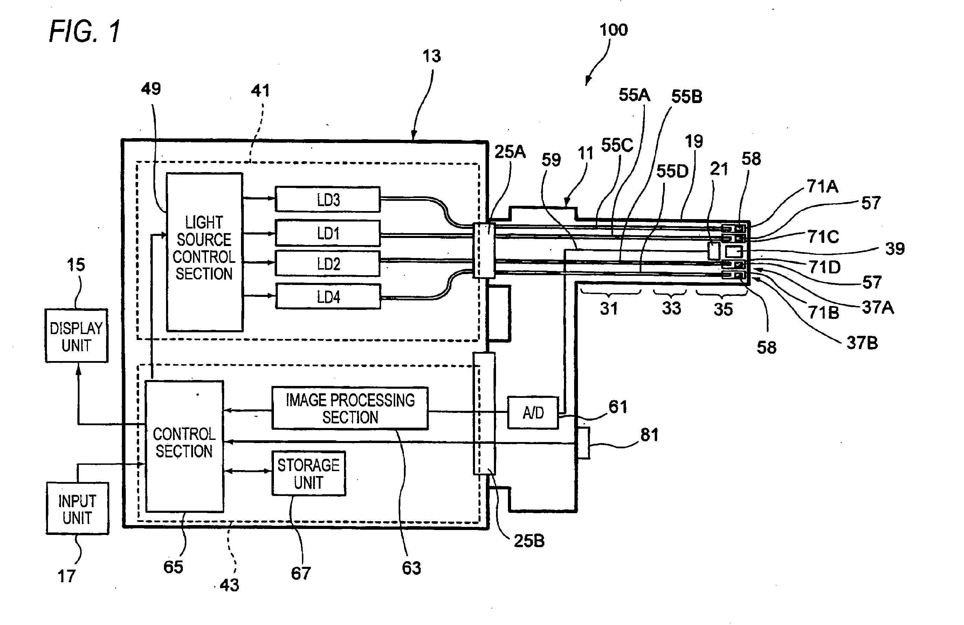

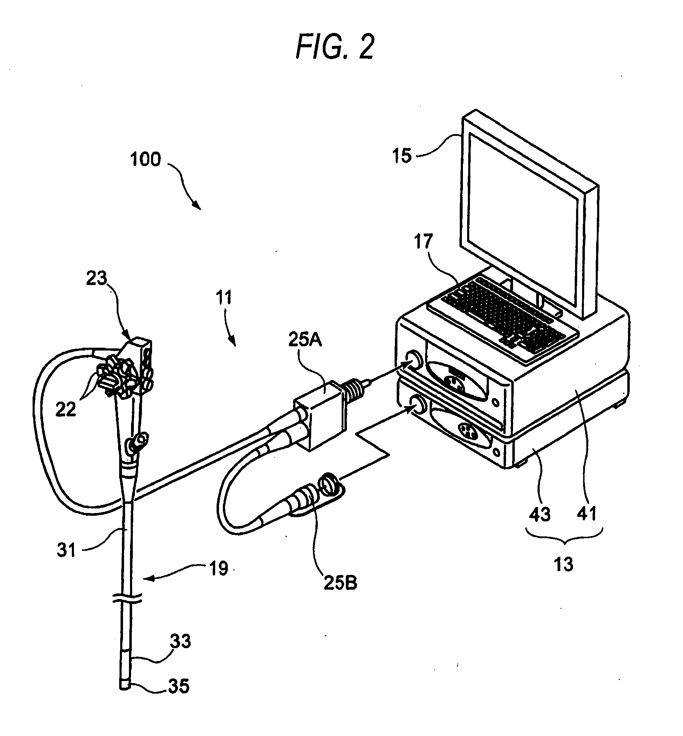

Fig. 1 is a conceptual block diagram showing the configuration of an endoscope apparatus according to the embodiment of the invention. Fig. 2 shows an appearance of an example of the endoscope apparatus of Fig. 1.

As shown in Figs. 1 and 2, the endoscope apparatus 100 as an example medical apparatus is equipped with an endoscope 11 and a control apparatus 13 to which the endoscope 11 is connected. A display unit 15 for displaying image information etc. and an input unit 17 for receiving an input manipulation are connected to the control apparatus 13. The endoscope 11 is an electronic endoscope which has an illumination optical system for emitting illumination light from a tip portion of an endoscope insertion unit 19 to be inserted into a subject body and an imaging optical system including an imaging device 21 (see Fig. 1) for imaging an observation region.

The endoscope 11 is equipped with the endoscope insertion unit 19, a manipulation unit 23 (see Fig. 2) for performing a curving the tip portion of the endoscope insertion unit 19 and a manipulation for observation, and connector units 25A and 25B which connect the endoscope 11 to the control apparatus detachably. Although not shown in Fig. 1 or 2, various channels such as a forceps channel through which to insert a tissue picking tool or the like and an air/water feed channel are formed inside the manipulation unit 23 and the endoscope insertion unit 19.

The endoscope insertion unit 19 is composed of a flexible soft portion 31, a bendable portion 33, a tip portion (hereinafter referred to as an endoscope tip portion) 35. As shown in Fig. 1, illumination windows 37A and 37B through which to illuminate an observation region with light and the imaging device 21 such as a CCD (charge-coupled device) image sensor or a CMOS (complementary metal-oxide-semiconductor) image sensor for acquiring image information from the observation region are arranged in the endoscope tip portion 35. An objective lens unit 39 to serve as an observation window is disposed adjacent to the photodetecting surface of the imaging device 21.

The bendable portion 33, which is located between the soft portion 31 and the tip portion 35, may be bent freely by a manipulation of rotating angle knobs 22 which are provided in the manipulation unit 23 (see Fig. 2). The bendable portion 33 may be bent to any direction at any angle according to a part or the like of a subject body to be observed by the endoscope 11, whereby the illumination windows 37A and 37B and the observing direction of the imaging device 21 of the endoscope tip portion 35 may be directed to a desired part to be observed. The structure of the illumination windows 37A and 37B of the endoscope insertion unit 19 will be described later in detail.

The control apparatus 13 is equipped with a light source apparatus 41 for generating illumination light to be supplied to the illumination windows 37A and 37B of the endoscope tip portion 35 and a processor 43 for performing image processing on an image signal that is supplied from the imaging device 21. The control apparatus 13 is connected to the endoscope 11 by the connector units 25A and 25B. The above-mentioned display unit 15 and the input unit 17 are connected to the processor 43. The processor 43 performs image processing on an image signal that is transmitted from the endoscope 11, generates a display image, and supplies it to the display unit 15 according to an instruction that is supplied from the manipulation unit 23 of the endoscope 11 or the input unit 17.

The light source apparatus 41 is equipped with plural laser light sources having different center wavelengths. In the configuration of Fig. 1, the light source apparatus 41 is equipped, as a basic set, with laser light sources LD1 and LD2 having a center wavelength 405 nm and laser light sources LD3 and LD4 having a center wavelength 445 nm and may additionally be equipped with laser light sources having other center wavelengths.

Dimming-controlled individually by a light source control section 49, the laser light sources LD1-LD4 may emit laser beams individually or simultaneously. The emission timing and the light quantity ratio of the laser light sources LD1-LD4 may be changed arbitrarily.

The laser light sources LD1 and LD2 are light sources for special light observation which emit violet laser light having a center wavelength 405 nm toward an observation region not through a phosphor (described later). The laser light sources LD3 and LD4 are light sources for ordinary observation which emit blue laser light having a center wavelength 445 nm and generate white illumination light using a phosphor as a wavelength conversion member (described later). High-luminance white light may be generated with high efficiency by blue laser light emitted from the laser light sources LD3 and LD4 and the phosphor.

Illumination light that is emitted from the laser light sources LD1 and LD2 is used as illumination light for narrow-band-light observation and fluorescence observation and may also be used as illumination light for photodynamic diagnosis (PDD). Photodynamic diagnosis is a diagnosing method of giving a photosensitive substance that has tumor affinity and is sensitive to particular excitation light to a living body in advance, illuminating the surface of living body tissue with laser light as excitation light, and observing fluorescence emitted from a part, containing the photosensitive substance at a high density, of a focus of a tumor of cancer or the like.

Switching between special light observation using the laser light sources LD1 and LD2 and ordinary observation using the laser light sources LD3 and LD4 may be made by manipulating a changeover switch 81 of the endoscope apparatus 100 or the input unit 17 or made by the light source apparatus 41 with arbitrary timing or programmed, prescribed timing. If the endoscope apparatus 100 is configured so that selection may be made between preset exit light quantity ratios by a switch manipulation or the like, switching between an ordinary observation image and a special light observation image may be made easily. Naturally, it is possible to display both images simultaneously.

Each of the laser light sources LD1-LD4 may be a broad-area-type InGaN laser diode, and may also be an InGaNAs laser diodes or a GaNAs laser diode. Alternatively, each light source may be another type of semiconductor light-emitting device such as a light-emitting diode or a light source that is not a semiconductor light-emitting device. For example, light obtained by performing wavelength selection using a color filter on light emitted from a white light source such as a xenon lamp may be used.

Laser beams emitted from the laser light sources LD1-LD4 are introduced into optical fibers through condenser lenses (not shown), respectively. The laser beams emitted from the laser light sources LD1 and LD2 are transmitted to optical fibers 55A and 55B via the connector unit 25A, and the laser light sources LD3 and LD4 are transmitted to optical fibers 55C and 55D via the connector unit 25A.

Each of the optical fibers 55A-55D is a multimode fiber and may be, for example, a small-diameter fiber cable in which the core diameter is 105 µm, the clad diameter is 125 µm, and the diameter of the cable including a protective layer (outer sheath) is 0.3 to 0.5 mm.

Laser beams emitted from the laser light sources LD1-LD4 are introduced, with arbitrary timing, into the optical fibers 55A-55D which extend from the connector unit 25A to the endoscope tip portion 35. Each of laser beams emitted from the laser light sources LD1 and LD2 is transmitted to a light deflecting/diffusing member 57 and resulting illumination light is emitted toward an observation region. Each of laser beams emitted from the laser light sources LD3 and LD4 is transmitted to a phosphor 58 disposed in the endoscope tip portion 35 and resulting white illumination light is emitted toward an observation region.

The optical fiber 55C and the associated phosphor 58 constitute a light output unit 71A, and the optical fiber 55D and the associated phosphor 58 constitute a light output unit 71B. The optical fiber 55A and the associated light deflecting/diffusing member 57 constitute a light output unit 71C, and the optical fiber 55B and the associated light deflecting/diffusing member 57 constitute a light output unit 71D.

The pair of light output units 71A and 71B are disposed on the two respective sides of the imaging device 21 and the objective lens unit 39 in the endoscope tip portion 35. Likewise, the pair of light output units 71C and 71D are disposed on the two respective sides of the imaging device 21 and the objective lens unit 39.

Next, the structures of the light output units 71A-71D which are provided in the endoscope tip portion 35 will be described.

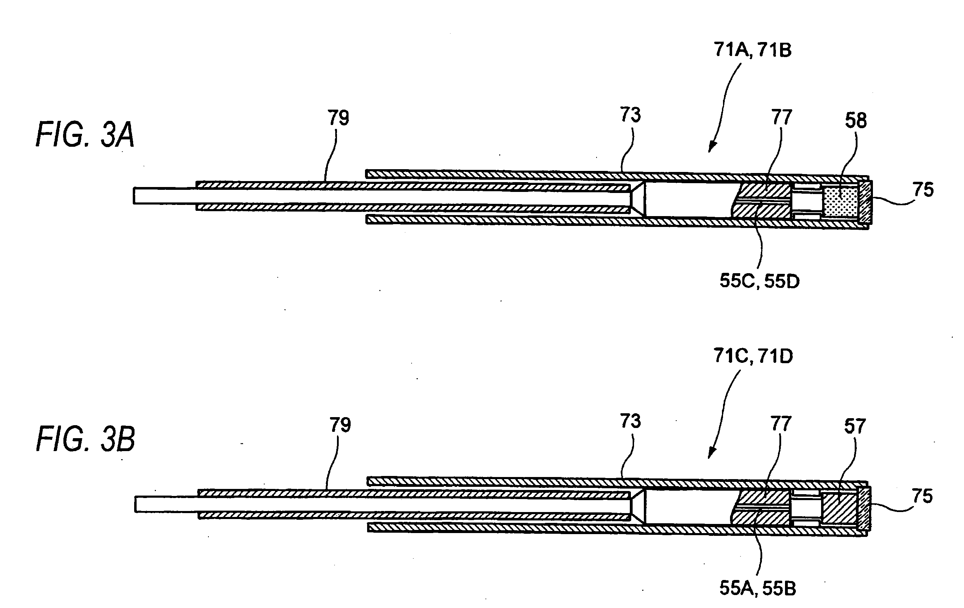

Fig. 3A is a sectional view showing the structure of the light output units 71A and 71B, and Fig. 3B is a sectional view showing the structure of the light output units 71C and 71D. The light output units 71A and 71B have the same structure which includes the phosphor 58, a cylindrical sleeve member 73 which surrounds the circumference of the phosphor 58, a protective glass (illumination window) 75 which seals the sleeve member 73 on one side, and a ferule 77 which is inserted in the sleeve member 73 and holds the optical fiber 55C or 55D so that it extends along the center axis. A flexible sleeve 79 is inserted between the sleeve member 73 and the outer surface of an outer sheath of that portion of the optical fiber 55C or 55D which extends from the rear end of the ferule 77.

On the other hand, the light output units 71C and 71D have the same structure which is the same as the structure of the light output units 71A and 71B except that the light deflecting/diffusing member 57 is provided in place of the phosphor 58 and light is introduced from the optical fiber 55A or 55B.

The phosphor 58 of each of the light output units 71A and 71B contains plural kinds of phosphor substances (e.g., YAG phosphor or BAM (BaMgAl₁₀O₁₇)) that are excited by absorbing part of blue laser light emitted from the laser light source LD3 or LD4 and emit green to yellow light. White (quasi-white) illumination light is generated as a combination of green to yellow light emitted through excitation by part of blue laser light and the other part of the blue laser light that passes through the phosphor 58 without being absorbed.

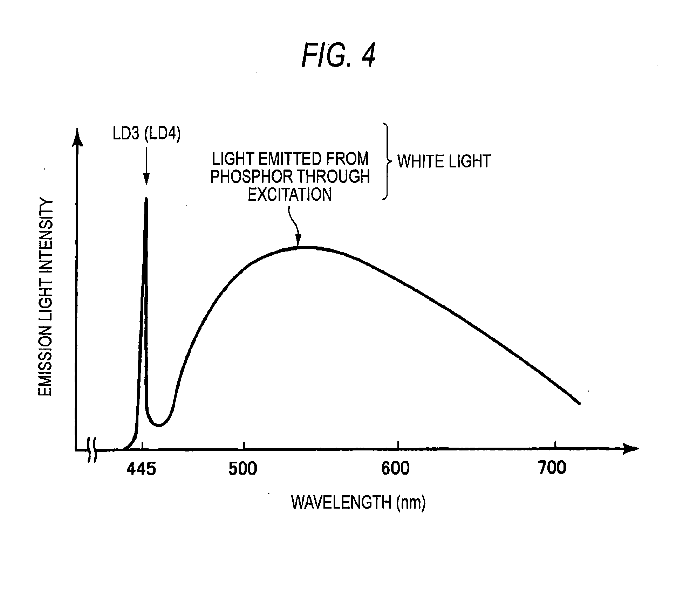

Fig. 4 is a graph showing emission spectra of blue laser light emitted from the laser light sources LD3 and LD4 and light generated through wavelength conversion of the blue laser light by the phosphor 58. The blue light is represented by an emission line having a center wavelength 445 nm, and the light emitted from the phosphor 58 through excitation by the blue laser light has a spectra light intensity profile whose wavelength band is approximately 450 to 700 nm. As mentioned above, the blue light and the light emitted through excitation having the above wavelength profiles form white light. Where a semiconductor light-emitting device is used as an excitation light source as in the example configuration being discussed, high-intensity white light may be generated with high emission efficiency, the intensity of white light may be adjusted easily, and variations in color temperature and chromaticity of white light may be made small.

The term "white light" as used in this specification is not limited to light having all wavelength components of visible light in a strict sense and may be light having particular wavelength components, for example, two or all of R (red), G (green), and B (blue) which are primary colors. In a broad sense, this time includes light having green to red wavelength components or blue to green wavelength components.

The phosphor 58 may prevent superimposition of imaging-obstructive noise or occurrence of a flicker at the time of display of a moving image which would otherwise be cause by a speckle pattern that is produced due to the coherence of laser light. The phosphor substances and a fixing/solidifying resin (filler) may have such particle diameters as to be low in absorption and high in scattering for infrared light taking their differences in refractive index into consideration. This enhances the scattering effect and lowers the optical loss without decrease in light intensity for red light or infrared light.

The light deflecting/diffusing member 57 of each of the light output units 71C and 71D may be made of a material that transmits laser light emitted from the laser light source LD1 or LD2, such as a transparent resin material or glass. The light deflecting/diffusing member 57 may be such that the surface of a member made of a resin material or glass is formed with minute asperities or a light diffusion layer containing particles (filler or the like) having a different refractive index. Or the light deflecting/diffusing member 57 may be made of a semi-transparent material. As a result, light that is transmitted from the light deflecting/diffusing member 57 is made light having a narrow wavelength band that is uniform in light quantity in a prescribed illumination region.

Returning to Fig. 1, white light that is a combination of blue laser light and light emitted from the phosphor 58 through excitation or narrow-band laser light is applied to an observation region of a subject body from the endoscope tip portion 35. An image (subject body image) of the observation region being illuminated with the illumination light is formed by the objective lens unit 39 (observation window) and imaged by the imaging device 21.

An image signal of an image taken that is output from the imaging device 21 after the imaging is transmitted via a scope cable 59 to an A/D converter 61, where it is converted into a digital signal. The digital signal is input to an image processing section 63 of the processor 43 via the connector unit 25B. The image processing section 63 performs various kinds of processing such as white balance correction, gamma correction, outline emphasis, and color correction on the digital signal that has been obtained by converting the image signal supplied from the imaging device 21. A resulting image signal and other various kinds of information are used by the control section 65 to produce an endoscope observation image, which is displayed on the display unit 15. If necessary, the endoscope observation image is stored in a storage unit 67 which is a memory or a storage device.

Next, example structures of the endoscope tip portion 35 will be described in detail.

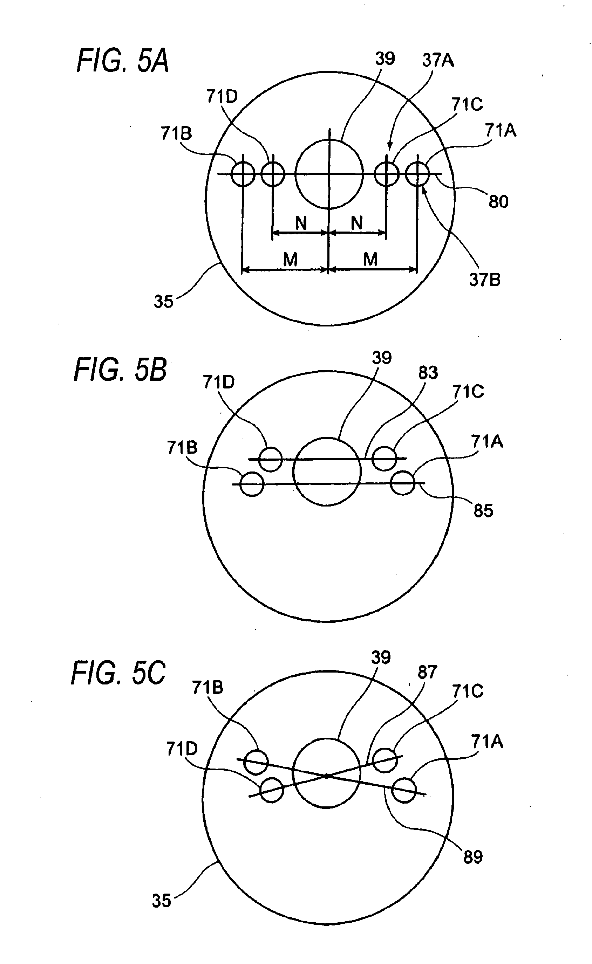

Fig. 5A is a front view of an endoscope tip portion 35 in which plural illumination windows are arranged in line, Fig. 5B is a front view of an endoscope tip portion 35 in which pairs of illumination windows for emitting light beams having the same spectrum are arranged on parallel lines, and Fig. 5C is a front view of an endoscope tip portion 35 in which pairs of illumination windows for emitting light beams having the same spectrum are arranged on intersecting lines.

In the tip portion 35 of the endoscope insertion unit 19 (see Figs. 1 and 2), illumination windows 37B which emit longer wavelength light are spaced from the objective lens unit 39 by a distance M. Illumination windows 37A which emit shorter wavelength light are spaced from the objective lens unit 39 by a distance N which is shorter than the distance M.

In the example structure of Fig. 5A, the illumination windows 37A correspond to the respective light output units 71C and 71D and the illumination windows 37B correspond to the respective light output units 71A and 71B. That is, the pair of light output units 71A and 71B which emit longer wavelength light are disposed on the two respective sides of the objective lens unit 39 at the same distance M, and the pair of light output units 71C and 71D which emit shorter wavelength light are disposed inside the light output units 71A and 71B on the two respective sides of the objective lens unit 39 at the same distance N. The light output units 71C and 71D emit shorter wavelength light having a center wavelength 405 nm, and the light output units 71A and 71B emit white light that is generated from laser light having a center wavelength 445 nm. The distances M between the objective lens unit 39 and the light output units 71A and 71B need not always be identical in a strict sense and may have such a difference as not to cause any substantial influence. Likewise, the distances N between the objective lens unit 39 and the light output units 71C and 71D need not always be identical in a strict sense and may have such a difference as not to cause any substantial influence.

In the structure of Fig. 5A, since light beams having the same spectrum are emitted from the two respective sides of the objective lens unit 39, an observation image is prevented from having shadows even if asperities exist in an observation region, which facilitates examination of the entire image. Furthermore, since shorter wavelength light beams are emitted from the positions that are closer to the objective lens unit 39 than the positions from which longer wavelength light beams are emitted, the shorter wavelength light beams, with which the light quantity tends to decrease to a larger extent as the illumination position comes closer to the illumination center position, may be applied from the positions that are near the illumination center position corresponding to the center of an observation image. With this measure, shorter wavelength light may be applied with a uniform light quantity distribution to a region from the illumination center position corresponding to the center of an observation image to positions corresponding to both ends of the observation image and shading may thus be prevented in close-proximity shooting.

As shown in Fig. 5A, the light output units 71A-71D are disposed on the same line 80 and the pair of light output units 71A and 71B and the pair of light output units 71C and 71D are each arranged so as to be point-symmetrical with respect to the objective lens unit 39. Therefore, light quantities on both sides of the objective lens unit 39 may be balanced and shading and color unevenness may be made less prone to occur. In particular, since the interval between the light input units 71A and 71B which emit white light is set large, occurrence of shades may be prevented more reliably.

As shown in Fig. 5B, the pair of light output units 71C and 71D for emitting light beams having the same spectrum and the pair of light output units 71A and 71B for emitting light beams having the same spectrum may be arranged on parallel lines 83 and 85, respectively. With this arrangement, the overall length of the arrangement of the light output units 71A-71D may be made shorter than in the linear arrangement and the endoscope diameter may be reduced.

As shown in Fig. 5C, the pair of light output units 71C and 71D for emitting light beams having the same spectrum and the pair of light output units 71A and 71B for emitting light beams having the same spectrum may be arranged on intersecting lines 87 and 89, respectively. With this arrangement, the overall length of the arrangement of the light output units 71A-71D may be made shorter (the arrangement may be made more compact) than in the linear arrangement. Furthermore, since the pair of light output units 71A and 71B and the pair of light output units 71C and 71D are each arranged point-symmetrically, light quantities on both sides of the objective lens unit 39 may be balanced.

As described above, in the endoscope apparatus 100 according to the embodiment, the light output units 71C and 71D which are dedicated to emission of shorter wavelength light, with which the light quantity tends to decrease to a larger extent as the illumination position comes closer to the illumination center position corresponding to the center of an observation image, are disposed close to the objective lens unit 39. The phenomenon that the light quantity decreases toward the center of the imaging surface may be suppressed. As a result, the amount of shorter wavelength light incident on the center of the imaging surface is made equal to the amount of longer wavelength light incident thereon, and shading and color unevenness may be prevented in close-proximity shooting.

The positional relationships between the light output units 71A and 71B, the light output units 71C and 71D, and the objective lens unit 39 are not limited to those in which the pair of light output units 71A and 71B and the pair of light output units 71C and 71D are each arranged so as to be point-symmetrical with respect to the objective lens unit 39. For example, an arrangement is possible in which only the light output unit 71A is spaced from the objective lens unit 39 by a distance M and only the light output unit 71C for emitting shorter wavelength light is spaced from the objective lens unit 39 by a distance N which is shorter than the distance M.

The objective lens unit 39 may be disposed on the line that connect the pair of light output units 71A and 71B or the pair of light output units 71C and 71D. However, the invention is not limited to such a case. The objective lens unit 39 may be disposed off the line that connect the pair of light output units 71A and 71B or the pair of light output units 71C and 71D as long as the light output units 71A and 71C are disposed in one of two regions obtained by equally dividing the tip surface of the endoscope tip portion 35 into two regions by a boundary line passing through the center of the objective lens unit 39 (observation window) and the light output units 71B and 71D are disposed in the other region. In this case, an observation image that would be taken with illumination light having a uniform distribution in an illumination region corresponding to the observation image could be obtained by performing shading correction on observation image data obtained by imaging when necessary.

The above-described endoscope apparatus 100 may be modified in the following manner. In addition to two kinds of light, that is, laser light having the center wavelength 405 nm and white light generated with excitation of the phosphor 58 by laser light having the center wavelength 445 nm, light having another wavelength component is emitted. For example, other illumination windows associated with a pair of light output units are disposed outside the illumination windows 37A and 37B on the two respective side of the objective lens unit 39 and even longer wavelength light having a center wavelength 785 nm, for example, is emitted from these illumination windows.

Next, a description will be made of the emission timing of the laser light sources LD1-LD4 which supply laser beams to the respective light output units 71A-71D.

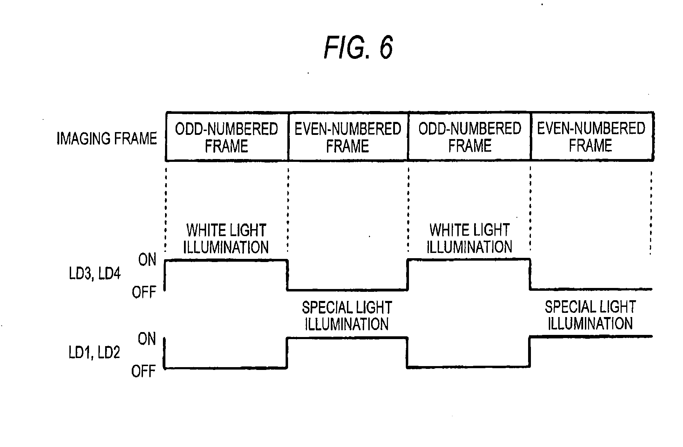

Fig. 6 shows an example manner of driving of the laser light sources LD1-LD4 and the imaging device 21 in the case where imaging is performed by using two kinds of illumination light having different center wavelengths. As described above, the laser light sources LD1 and LD2 emit laser light (special light) having the center wavelength 405 nm and the laser light sources LD3 and LD4 emit laser light having the center wavelength 445 nm. The laser light emitted from each of the laser light sources LD3 and LD4 is wavelength-converted by the phosphor 58, and white light is emitted from each of the light output units 71A and 71B.

Even for the same observation region, an observation image taken with white light illumination and an observation image taken with special light illumination at the particular wavelength are different from each other because of different observation subjects. Therefore, when displayed on the display unit 15 shown in Fig. 1, the display form of each observation image is set arbitrarily so as to facilitate acquisition of desired information. For example, switching is made between the white light illumination and the special light illumination in synchronism with imaging frames of the imaging device 21 so that the white light illumination and the special light illumination are employed in odd-numbered frames and even-numbered frames, respectively.

In this case, an observation image of ordinary observation and an observation image of special light observation may be recognized simultaneously in a state that they are registered with each other by displaying, in superimposition, as a single piece of image information, information of even-numbered frames taken with the special light illumination and information of odd-numbered frames taken with the white light illumination. As a result, recognition of an observation region with the white light illumination and observation of a feature portion with the special light illumination may be made more reliably with high legibility. There is another advantage that superimposing a white light observation image on a special light observation image enhances color reproduction performance and thereby enables display of a more natural image.

Instead of superimposing an image of even-numbered frames and an image of odd-numbered frames to produce a single piece of image information, they may be displayed at different positions in the display area of the display unit 15. In this case, observation and treatment may be done while comparing the two images.

Next, a description will be made of modifications in which LED light sources are used in place of laser light sources.

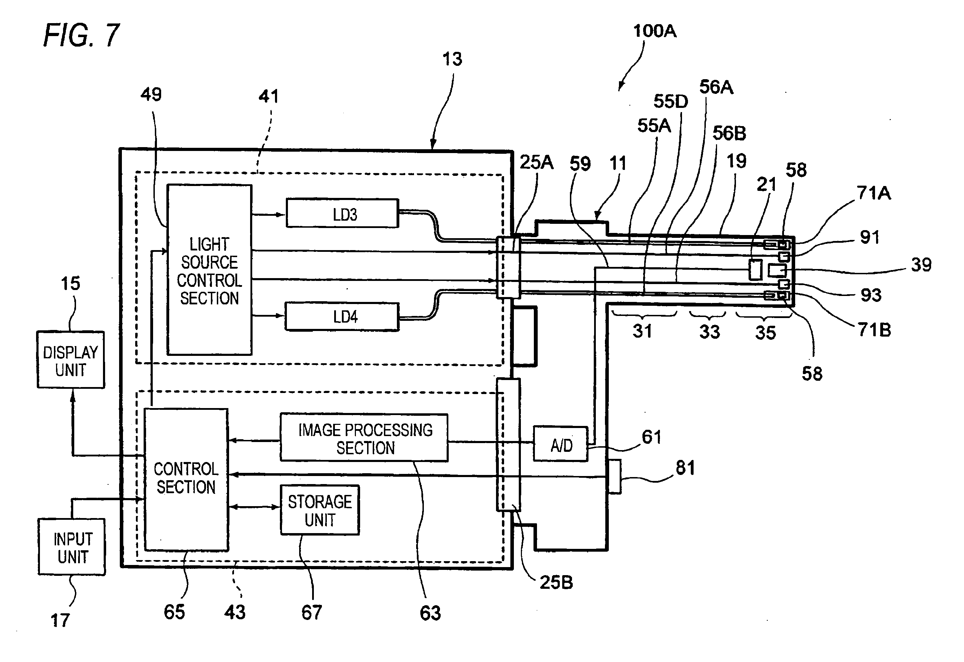

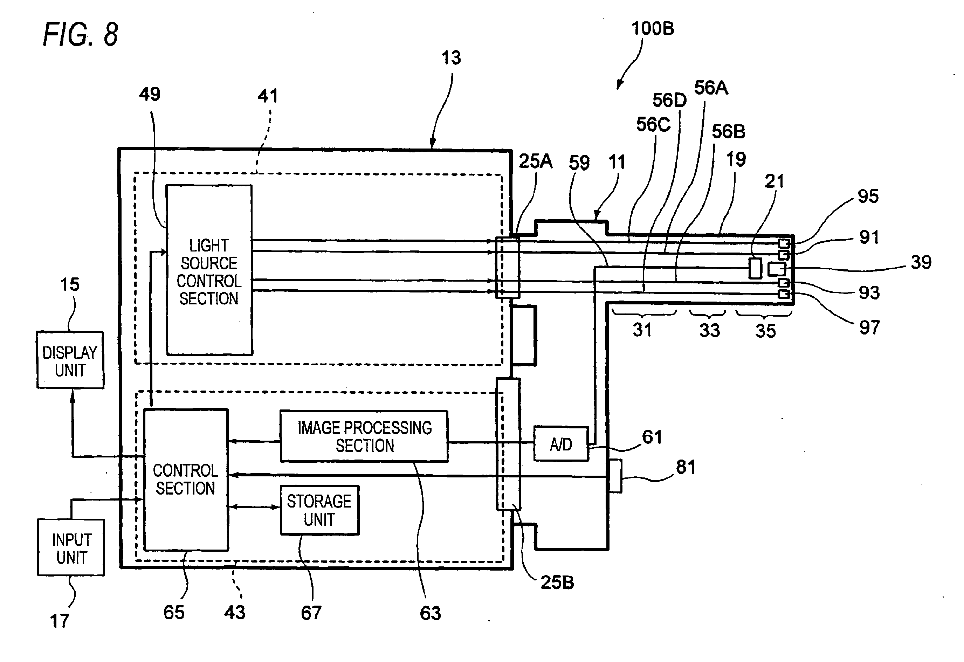

Figs. 7 and 8 are block diagrams showing the configurations of endoscope apparatus in which LEDs are used as light output units.

Whereas in the endoscope apparatus 100 of Fig. 1 the laser light sources LD1-LD4 are used as the light sources for supplying light beams to the light output units 71A-71D, at least part of the sets of a laser light source and a light output unit may be replaced by light-emitting diodes. In the modified endoscope apparatus 100A of Fig. 7, the laser light sources LD3 and LD4 are used as the light sources of the light output units 71A and 71B and light-emitting diodes 91 and 93 are used in place of the combination of the laser light source LD 1and the light output unit 71C and the combination of the laser light source LD2 and the light output unit 71D, respectively. For example, the light-emitting diodes 91 and 93 are attached to support bodies of tip hard portions that are located inside the illumination windows 37A.

The light-emitting diodes 91 and 93 are violet light-emitting diodes having a center wavelength 405 nm and have a function of emitting special light like the above-described light output units 71C and 71D. The light-emitting diodes 91 and 93 are connected to the light source control section 49 by signal lines 56A and 56B, and the emission timing of the light output units 71A and 71B and the light-emitting diodes 91 and 93 may be changed individually by the light source control section 49 and the light quantity ratio between the light output units 71A and 71B may be changed arbitrarily by the light source control section 49. The light quantity ratio between the light-emitting diodes 91 and 93 may also be changed arbitrarily, that is, the light quantity ratio may be adjusted to an optimum value (to 1 or a desired value) according to an illumination subject.

In the endoscope apparatus 100A, since the light-emitting diodes 91 and 93 are used as the light sources for special light observation, the light sources may be elongated in life and reduced in power consumption. The light sources themselves may be reduced in cost and weight, and the maintenance cost may also be reduced.

The modified endoscope apparatus 100B of Fig. 8 is different from the modified endoscope apparatus 100A of Fig. 7 in that light-emitting diodes 95 and 97 are used in place of the combination of the laser light source LD3 and the light output unit 71A and the combination of the laser light source LD4 and the light output unit 71B of the latter, respectively. The light-emitting diodes 95 and 97 are white light-emitting diodes each of which consists of, for example, a blue light-emitting diode and a phosphor for converting blue light emitted from the blue light-emitting diode into yellow light. In each of the light-emitting diodes 95 and 97, part of blue light emitted from the blue light-emitting diode is absorbed by the phosphor and wavelength-converted into yellow light and white light is output as a combination of the yellow light and the other (i.e., non-absorbed) part of the blue light. However, the structure of each of the light-emitting diodes 95 and 97 is not limited to the above, and white light may be emitted by a white light-emitting diode having another structure.

The light-emitting diodes 95 and 97 are connected to the light source control section 49 by signal lines 56C and 56D, and the emission timing of the light-emitting diodes 91, 93, 95, and 97 may be changed individually by the light source control section 49 and the light quantity ratio between the light-emitting diodes 91 and 93 or 95 and 97 may be changed arbitrarily by the light source control section 49.

In the endoscope apparatus 100B, all illumination light beams emitted from the endoscope tip portion 35 are generated by the light-emitting diodes 91, 93, 95, and 97. Therefore, the light sources may further be elongated in life and reduced in power consumption, cost, and weight.

As another modification, red light-emitting diodes, green light-emitting diodes, and blue light-emitting diodes which emit red light, green light, and blue light, respectively, may be disposed in the endoscope tip portion 35. In this case, the light-emitting diodes whose emission wavelength is shorter are disposed closer to the observation window. That is, the blue light-emitting diodes, the green light-emitting diodes, and the red light-emitting diodes are arranged in this order from inside on both sides of the observation window. With this structure, variations in a light quantity distribution for each wavelength component may be made small and hence color unevenness may be prevented. Uniform illumination light that is free of shading and color unevenness may thus be generated and good observation images may always be obtained stably. Optimum illumination light may be generated in a desired manner (e.g., hue adjustment is made or a light having a particular wavelength is emitted) according to a purpose of observation by independently controlling the light quantities of the pairs of light-emitting diodes of the respective colors.

As described above, in each of the endoscope apparatus 100, 100A, and 100B, the illumination windows 37A of shorter wavelength illumination light are disposed closer to the imaging device 21 than the illumination windows 37B of longer wavelength illumination light, the amount of shorter wavelength light incident on the objective lens unit 39 is made equal to the amount of longer wavelength light incident thereon. The wavelength dependence of a light quantity distribution may be made low even in close-proximity shooting in which the angle of view of the endoscope is about 50° to 60°. As a result, shading and color unevenness may be prevented from occurring in an observation image, which contributes to increase in the accuracy of a medical examination and a diagnosis using an endoscope. Furthermore, light quantity unevenness may be reduced in special light observation (e.g., narrow-band-light observation in which surface blood vessels etc. are emphasized and fluorescence observation in which autofluorescence or drug fluorescence is observed).

The invention is not limited to the above embodiment, and modifications and applications that would be made by a person skilled in the art on the basis of the disclosure of this specification and known techniques are included in the range of protection of the invention.

This specification discloses the following items.

(1) According to an aspect of the invention, a medical apparatus includes a plurality of illumination windows which are disposed in a tip portion of an insertion unit to be inserted into a subject body and which emit light beams supplied from a plurality of light sources and having different spectra toward the subject body, the plurality of illumination windows include: a pair of first illumination windows; and a pair of second illumination windows which are disposed inside the pair of first illumination windows, respectively, and emit light beams having a shorter wavelength than light beams emitted from the pair of first illumination windows; and an observation window through which to observe the subject body.

In this medical apparatus, since light having a shorter center wavelength may be emitted from positions that are closer to the observation window than positions from which light having a longer center wavelength is emitted, the shorter wavelength light, with which the light quantity tends to decrease to a larger extent as the illumination position comes closer to the illumination center position, may be applied from the positions that are near the illumination center position corresponding to the center of an observation image. As a result, shorter wavelength light may be applied with a uniform light quantity distribution to a region from the illumination center position corresponding to the center of an observation image to positions corresponding to both ends of the observation image and shading may thus be prevented in close-proximity shooting or the like.

(2) The medical apparatus of (1), wherein one of the pair of first illumination windows and one of the pair of second illumination windows are disposed in one of two regions obtained by equally dividing a tip surface of the tip portion of the insertion unit into two regions by a boundary line passing through a center of the observation window, and the other of the pair of first illumination windows and the other of the pair of second illumination windows are disposed in the other region.

In this medical apparatus, the pair of first illumination windows and the pair of second illumination windows (two pairs in total) are each disposed in the two respective regions located on both sides of the boundary line. With this measure, an observation region may be observed through the observation window in such a manner as to be illuminated almost uniformly with light beams emitted from a pair of illumination windows of the same kind. Therefore, even if asperities exist in the observation region, shades are prevented from occurring in an observation image.

(3) In the medical apparatus of (1) or (2), the pair of first illumination windows and the pair of second illumination windows are each disposed with the observation window interposed in between.

In this medical apparatus, since light beams are emitted simultaneously from both sides of the observation window, an observation region may be illuminated with a higher degree of uniformity.

(4) In the medical apparatus (1) to (3), the pair of first illumination windows emit light beams having the same spectrum, and the pair of second illumination windows emit light beams having the same spectrum.

In this medical apparatus, since each pair of illumination windows emit light beams having the same spectrum, light beams of the same kind are emitted from the illumination windows located on both sides of the observation window and an observation region may be illuminated uniformly.

(5) The medical apparatus of (1) to (4), wherein the pair of first illumination windows emit white illumination light beams, and the pair of second illumination windows emit light beams which are shorter in center wavelength than the white illumination light beams.

In this medical apparatus, the outside first illumination windows emit white illumination light and the inside second illumination windows emit light that is shorter in center wavelength than the white illumination light. As a result, shades are prevented from being formed in an observation region by white illumination light and the observation region may be illuminated uniformly by shorter wavelength light. Therefore, illumination unevenness may be prevented in each of an ordinary observation image and a special light observation image.

(6) In the medical apparatus of (1) to (5), the pair of first illumination windows and the pair of second illumination windows are each disposed on two respective sides of the observation window at approximately the same distance from the center of the observation window.

In this medical apparatus, since each pair of illumination windows are disposed at approximately the same distance from the observation window, illumination may be performed symmetrically with respect to the observation window and hence a light quantity distribution that is higher in the degree of uniformity may be obtained.

(7) According to an aspect of the invention, an endoscope apparatus which is a medical apparatus of (1) to (6), wherein a plurality of illumination windows and an observation window are disposed in a tip portion of an insertion unit of an endoscope to be inserted into the subject body.

This endoscope apparatus may emit illumination light having a uniform light quantity distribution and may produce a high-quality observation image because of absence of illumination unevenness.

(8) In the endoscope apparatus according to (7), a plurality of light sources include a light source having a laser light-emitting device as a light emission source.

This endoscope apparatus may emit high-intensity light with high efficiency.

(9) The endoscope apparatus of (8) further may comprises: laser light sources; optical fibers which guides laser beams emitted from the laser light sources to the tip portion of the insertion unit of the endoscope, respectively; and wavelength conversion members which are disposed in emission end portions of the optical fibers and wavelength-convert the laser beams, respectively, wherein
a pair of first illumination windows emit white light beams which are combinations of the laser beams and wavelength-converted light beams, respectively.

In this endoscope apparatus, white light is generated by exciting a phosphor (wavelength conversion member) by laser light having a wavelength 445 nm, for example. As a result, the scattering effect may be enhanced without lowering the light intensity, which allows ordinary observation to be performed with high efficiency using light that exhibits high color rendering performance. A good observation image may thus be obtained.

(10) In the endoscope apparatus of (7), the plurality of light sources include a light source having a light-emitting diode as a light emission source.

Because of the use of the light-emitting diode which is has a long life and is highly efficient and inexpensive, this endoscope apparatus may attain cost reduction and power saving.

(11) In the endoscope apparatus of (10), the light-emitting diode is disposed in the tip portion of the insertion unit of the endoscope.

In this endoscope apparatus, since the light-emitting diode is disposed in the tip portion of the insertion unit of the endoscope, the configuration of the endoscope apparatus may be simplified.

(12) The endoscope apparatus of (7) to (11) further may include a light source control section which changes at least one of a light emission timing relationship and an emission light quantity ratio of light sources that supply light beams to the pair of first illumination windows and light sources that supply light beams to a pair of second illumination windows.

In this endoscope apparatus, since light emitted from each illumination window may be controlled in a desired manner by the light source control section, illumination light that is suitable for a purpose of observation may be generated easily.

## Claims

1. A medical apparatus comprising:
a plurality of illumination windows which are disposed in a tip portion of an insertion unit to be inserted into a subject body and which emit light beams supplied from a plurality of light sources and having different spectra toward the subject body, the plurality of illumination windows include:
a pair of first illumination windows; and
a pair of second illumination windows which are disposed inside the pair of first illumination windows, respectively, and emit light beams having a shorter wavelength than light beams emitted from the pair of first illumination windows; and
an observation window through which to observe the subject body.

2. The medical apparatus according to claim 1, wherein one of the pair of first illumination windows and one of the pair of second illumination windows are disposed in one of two regions obtained by equally dividing a tip surface of the tip portion of the insertion unit into two regions by a boundary line passing through a center of the observation window, and the other of the pair of first illumination windows and the other of the pair of second illumination windows are disposed in the other region.

3. The medical apparatus according to claim 1 or 2, wherein the pair of first illumination windows and the pair of second illumination windows are each disposed with the observation window interposed in between.

4. The medical apparatus according to any one of claims 1 to 3, wherein the pair of first illumination windows emit light beams having the same spectrum, and the pair of second illumination windows emit light beams having the same spectrum.

5. The medical apparatus according to any one of claims 1 to 4, wherein the pair of first illumination windows emit white illumination light beams, and the pair of second illumination windows emit light beams which are shorter in center wavelength than the white illumination light beams.

6. The medical apparatus according to any one of claims 1 to 5, wherein the pair of first illumination windows and the pair of second illumination windows are each disposed on two respective sides of the observation window at approximately the same distance from the center of the observation window.

7. An endoscope apparatus which is a medical apparatus according to any one of claims 1 to 6, wherein a plurality of illumination windows and an observation window are disposed in a tip portion of an insertion unit of an endoscope to be inserted into the subject body.

8. The endoscope apparatus according to claim 7, wherein a plurality of light sources include a light source having a laser light-emitting device as a light emission source.

9. The endoscope apparatus according to claim 8, further comprising: laser light sources; optical fibers which guides laser beams emitted from the laser light sources to the tip portion of the insertion unit of the endoscope, respectively; and wavelength conversion members which are disposed in emission end portions of the optical fibers and wavelength-convert the laser beams, respectively, wherein
a pair of first illumination windows emit white light beams which are combinations of the laser beams and wavelength-converted light beams, respectively.

10. The endoscope apparatus according to claim 7, wherein the plurality of light sources include a light source having a light-emitting diode as a light emission source.

11. The endoscope apparatus according to claim 10, wherein the light-emitting diode is disposed in the tip portion of the insertion unit of the endoscope.

12. The endoscope apparatus according to any one of claims 7 to 11, further comprising a light source control section which changes at least one of a light emission timing relationship and an emission light quantity ratio of light sources that supply light beams to the pair of first illumination windows and light sources that supply light beams to a pair of second illumination windows.
